# FASCICULE DE BREVET EUROPEEN

(11) **EP 2 522 255 B1**
(45) Date de publication et mention de la délivrance du brevet: **19.03.2014**
(21) Numéro de dépôt: 12167321.4
(22) Date de dépôt: 09.05.2012
(51) Int. Cl.: A47C 21/04, A47C 27/08, A47C 31/00, A61G 7/057

(54) **Dispositif de régulation de l'humidité et de la température à la surface d'un élément de support**
Vorrichtung zur Feuchtigkeits- und Temperaturregulierung auf der Oberfläche eines Halterungselements
Device for controlling the humidity and temperature on the surface of a support element

(30) Priorité: 12.05.2011 FR 1154099
(43) Date de publication de la demande: 14.11.2012
(73) Titulaire: Hill-Rom Industries SA, 34100 Montpellier (FR)
(72) Inventeur: Gazagnes, Laetitia, 34080 MONTPELLIER (FR)
(74) Mandataire: Domange, Maxime

(56) Documents cités:
- EP-A1- 1 915 978
- WO-A1-2005/004779
- US-A- 5 290 904

## Description

La présente invention concerne des dispositif et procédé de régulation de l'humidité et de la température à la surface d'un élément de support du type matelas ou coussin et au voisinage et contact du corps d'un individu y reposant, dans le domaine technique des dispositifs de support de type matelas ou coussins pour supporter le corps d'une personne assise ou allongée sur le dispositif de support, et notamment des dispositifs de support de type matelas thérapeutiques pour supporter le corps de patients.

L'invention concerne plus particulièrement une housse de matelas thérapeutique sur lequel un individu peut être allongé dans des conditions de confort thermique et de contrôle de l'humidité optimales, cette housse assurant une gestion du microclimat optimal pour le patient, cette housse assurant une régulation du microclimat dans des conditions de température optimale pour le patient.

Cette humidité peut provenir du corps du patient par écoulement d'un liquide corporel tel que par sudation, ou d'une source externe de liquide répandu. Il est souhaitable en effet d'éviter la macération de liquide au niveau des tissus mous de la peau, qu'il s'agisse de liquide externe ou de transpiration, car cette humidification entraîne une macération laquelle favorise la formation d'escarres et entretient des foyers infectieux.

On connaît des procédé et dispositif de ce type consistant à injecter de l'air à la surface ou vers le corps de l'individu, en mettant en oeuvre des matelas ou coussins dits à perte d'air (« low-air-loss bed ») constitués de compartiments gonflés d'air sous pression. Ainsi, le corps de l'individu ou la zone entre le corps et l'élément de support, c'est-à-dire le matelas au niveau duquel peut créer une humidité, se trouve asséché par le flux d'air orienté dans cette direction.

Un premier problème de ce dispositif connu est qu'il ne peut pas être mis en oeuvre indépendamment de l'élément support, notamment du matelas et que l'interruption de l'injection d'air à l'intérieur du matelas conduit à le rendre inopérationnel. Un autre inconvénient de ce système de matelas dit à perte d'air est qu'il peut conduire à un assèchement excessif du corps et nécessite une compensation des pertes hydriques par un programme d'hydratation de l'individu.

On a décrit des systèmes de déshumidification au voisinage d'un patient constitués d'une housse comprenant une enveloppe intercalée entre le patient et le matelas, ladite enveloppe comprenant une couche supérieure et une couche inférieure délimitant une chambre dans laquelle on fait circuler de l'air. Dans ces systèmes, on cherche à déshumidifier la surface externe de ladite couche supérieure sur laquelle repose ledit patient, au moins en partie par transfert de vapeur d'eau par migration moléculaire des molécules d'eau à travers la couche supérieure de l'enveloppe perméable à la vapeur d'eau.

Dans US 5.882.349, la couche inférieure de l'enveloppe est imperméable à l'air et à la vapeur d'eau, et l'air est injecté le cas échéant dans une partie seulement du volume interne de ladite enveloppe par une pluralité d'orifices d'injection, et est évacué par une pluralité de perforations pouvant être disposées sur les côtés de la couche supérieure. Ce système de déshumidification est relativement peu performant avec une déshumidification annoncée de seulement 400 ml/24h.

Dans US 5.926.884, on a décrit une housse de matelas de ce type dans lequel l'air est évacué exclusivement par des perforations dans la couche supérieure sur toute la surface et notamment au niveau de la zone recouverte par le patient, et la couche inférieure est perméable à la vapeur d'eau. L'enveloppe ainsi formée entre la couche inférieure et la couche supérieure toutes deux perméables à la vapeur d'eau est complétée par une couche sous-jacente additionnelle absorbant et dispersant la vapeur d'eau risquant de s'accumuler entre le dispositif de déshumidification ainsi formé et le matelas.

Dans ces dispositifs de déshumidification par transfert de vapeur d'eau, l'évacuation d'air en partie au moins du côté du patient reposant sur le dispositif présente des risques de contamination de l'enveloppe par pénétration de liquide ou autre contaminant venant de la surface externe de la couche supérieure sur laquelle repose le patient. D'autre part, et surtout, les rendements en terme de déshumidification sont soit relativement faibles, soit s'accompagnent d'une déshydratation du patient résultant du flux d'air excessif envoyé à proximité du patient.

Dans EP 1 915 978 au nom de la demanderesse, on décrit un nouveau procédé et dispositif de régulation de l'humidité à la surface ou au voisinage du corps d'un individu reposant sur un élément support de type matelas ou coussin qui ne présentent pas les inconvénients ci-dessus et plus performant en termes de rendement de déshumidification.

Plus particulièrement, dans EP 1 915 978, le dispositif et procédé peut être mis en oeuvre indépendamment dudit élément de support et notamment sur tout type de matelas à air ou en mousse, ou autre, qui présente toutes les garanties de salubrité au regard des risques de contamination d'un fluide venant de l'extérieur, et qui ne nécessite pas la mise en oeuvre d'une hydratation concomitante du patient.

Ce dispositif de EP 1 915 978 permet une régulation de l'humidité automatisée et non pas simplement un dispositif permettant seulement de diminuer continûment l'humidité comme les dispositifs de matelas à perte d'air de la technique antérieure.

Plus précisément, dans EP 1 915 978 on fournit un dispositif de régulation de l'humidité à la surface d'un élément de support du type matelas ou coussin et au voisinage du corps d'un individu y reposant, comprenant une enveloppe formée par au moins deux pièces reliées entre elles au niveau de leurs bords périphériques, de préférence scellés par soudage, définissant une chambre intérieure, lesdites deux pièces consistant dans une première pièce ou pièce supérieure, destinée à être disposée du côté dudit corps de l'individu, et une deuxième pièce ou pièce inférieure, destinée à être disposée du côté dudit élément de support de type matelas ou coussin, ladite première pièce étant constituée d'un matériau formant une barrière imperméable à l'air et à l'eau liquide et perméable à la vapeur d'eau, ladite deuxième pièce étant constituée d'un matériau perméable à la vapeur d'eau, ladite deuxième pièce comprenant au moins un orifice d'injection d'air et des moyens d'évacuation d'air comprenant des zones perforées ou poreuses perméables à l'air, de préférence des perforations.

On comprend que ladite première pièce est non perforée et que l'air s'évacue exclusivement à travers ladite deuxième pièce intercalée entre ladite première pièce et ledit élément de support du type matelas ou coussin.

Dans le cas d'un matelas disposé horizontalement sur un lit, ladite première pièce constitue une pièce supérieure sur laquelle repose le corps de l'individu et ladite deuxième pièce constitue une pièce inférieure appliquée sur le matelas et disposée dessous ladite première pièce ou pièce supérieure.

Un dispositif tel que décrit dans EP 1 915 978 apporte un contrôle de l'humidité satisfaisant, notamment lorsqu'il est utilisé sur des matelas thérapeutiques pour les soins des patients. Toutefois, il présente une limite d'efficacité thermique car la courbe mesurée des températures de la peau du patient alité sur ces dispositifs augmente avec le temps et dépasse la température critique de confort de 35°C, au- delà de laquelle le patient alité se trouve dans un état de risque de l'apparition d'escarres de décubitus dans des zones du corps à risque, telles que les zones du sacrum et celles des talons par exemple.

Un autre problème est que certains patients ont des baisses de température, notamment dans certaines pathologies, en-dessous de la température limite inférieure de 28°C, en dessous de laquelle la santé du patient est en danger.

Le but de la présente invention est donc de fournir un dispositif et procédé qui permet tout à la fois le contrôle de l'humidité du patient au niveau de la zone de contact avec le matelas sur lequel il repose d'une part, et, d'autre part, le contrôle de la température de la peau du patient en contact avec le matelas, de façon à ce que celle-ci ne dépasse pas une température limite supérieure de préférence de 35°C, qui est la température limite de confort et ne descende pas en dessous d'une température limite inférieure, de préférence de 28°C, qui est la température limite basse de confort. Le dispositif selon l'invention permet ainsi de réduire l'apparition d'escarres qui résultent de la combinaison d'humidité et de température excessives mentionnées ci-dessus.

On connaît des matériaux à changement de phase ci-après dénommés matériaux PCM, plus particulièrement des tissus revêtus de microcapsules de matériaux PCM, dénommés ci-après tissus PCM (commercialisés par exemple par les sociétés Outlast Technology (USA) ou Schoeller Textil (Suisse). Ces tissus sont utilisés dans les vêtements pour protéger contre l'excédent de chaleur en absorbant la chaleur lorsque le corps en produit trop ou en la restituant lorsque le corps est refroidi. De façon connue, les microcapsules de matériaux PCM peuvent être intégrées à l'intérieur du tissu et même à l'intérieur des fibres constitutives du tissu ou revêtus à la surface du tissu dans un enduit. Cette technologie a été développée à l'origine pour la NASA afin de protéger les astronautes des fluctuations de températures importantes dans l'espace.

Les inventeurs ont essayé de mettre en oeuvre un tissu PCM de ce type juxtaposé par-dessus un dispositif de régulation d'humidité tel que décrit ci-dessus. Toutefois, ils ont observé que cette disposition apparemment évidente et optimale pour réduire la température, affectait non seulement l'efficacité de la régulation de la diminution d'humidité au contact du corps du patient, mais en outre était moins efficace pour limiter la montée en température du patient au niveau des zones de contact avec le dispositif selon l'invention.

En fait, les inventeurs ont découvert que, de façon surprenante, la mise en oeuvre optimale d'un tissu PCM en combinaison avec un dispositif de contrôle et de régulation de l'humidité à des fins de contrôle et de régulation de l'humidité et de la température, nécessitait de mettre en oeuvre le tissu PCM dans des conditions particulières d'exposition à un flux d'air lesquelles conditions n'étaient pas satisfaites en cas de contact direct du tissu PCM avec le corps du patient, intercalé entre le patient et le dispositif de régulation de l'humidité tel que décrit ci-dessus.

Plus précisément, la présente invention fournit un dispositif de régulation de l'humidité et de la température à la surface d'un élément de support du type matelas ou coussin et au voisinage et contact du corps d'un individu y reposant, comprenant une enveloppe formée par au moins deux pièces de préférence reliées entre elles au niveau de leurs bords périphériques, de préférence encore scellés par soudage, définissant une chambre intérieure, lesdites deux pièces consistant dans une première pièce destinée à être disposée du côté dudit corps de l'individu, et une deuxième pièce destinée à être disposée du côté dudit élément de support de type matelas ou coussin, ladite première pièce étant constituée d'un matériau formant une barrière imperméable à l'air et à l'eau liquide et perméable à la vapeur d'eau, ladite deuxième pièce étant constituée d'un matériau perméable à la vapeur d'eau, ladite deuxième pièce comprenant au moins un orifice d'injection d'air et des moyens d'évacuation d'air comprenant des zones perforées ou poreuses perméables à l'air, de préférence des perforations, **caractérisé en ce que** ladite chambre intérieure comprend des capsules de matériau(x) à changement de phase (PCM) dont la température de cristallisation la plus basse est supérieure à 25°C, de préférence supérieure à 28°C, et la température de fusion la plus élevée est inférieure à 40°C, de préférence inférieure à 35°C, lesdites capsules étant intégrées au sein et/ou disposées en surface d'un support, de préférence un support constitué d'un matériau fibreux tissé ou non-tissé, perméable à l'air.

Plus particulièrement, les microcapsules de matériau PCM présentent un diamètre de 5 µm à 5 mm.

On comprend que les capsules de matériau PCM sont réparties dans et/ou à la surface dudit support perméable à l'air au moins en regard des zones de contact du corps avec le dispositif selon l'invention de manière à ce que au moins lesdites zones de contact soient régulées en l'humidité et en température, à savoir les zones de contact des parties suivantes du corps : la tête, la partie dorsale, la zone du sacrum et les talons, lorsqu'un patient est allongé par-dessus un dispositif selon l'invention, ce dernier reposant sur un matelas.

Lorsque le dispositif selon l'invention est en fonctionnement, le flux d'air circule à l'intérieur entre la pièce inférieure et la pièce supérieure ; les capsules de PCM étant initialement en phase solide dans les conditions normales de température et de pression (25°C, 1 atm).

Lorsque le patient est alité depuis quelques minutes, la température de la surface de sa peau va avoir tendance à augmenter à cause d'une accumulation de la chaleur. Ainsi, cette température de la peau va augmenter et tendre vers des températures d'inconfort au-delà de 35°C ; alors, le patient étant en contact indirect avec les capsules PCM dont les températures de changement de phase se situe entre 20 et 40°C, les PCM à son contact vont changer d'état en passant de l'état solide à l'état liquide si leur température atteint la température de fusion la plus élevée définie ci-dessus. En effet, ce changement de phase de la matière nécessite de l'énergie, appelée « chaleur latente » portée par la chaleur dégagée par le patient, et utilisera l'énergie à disposition, cet excès de chaleur crée par le patient. La température de la peau du patient va donc se stabiliser dans une zone de confort ne dépassant pas la température limite définie ci-dessus, car l'excès de chaleur est supprimé.

Grâce à ces capsules de matériau PCM en combinaison avec un flux d'air à l'intérieur de ladite chambre, un gradient de température se crée entre le patient et l'intérieur du dispositif, entraînant un transfert spontané de la chaleur, du patient vers l'intérieur du dispositif.

Inversement, si la température de la peau du patient diminue à cause d'une pathologie et descend en dessous de la zone de confort (inférieure à 25°C, de préférence 28°C), les PCM en contact indirect avec le patient, repassent de l'état liquide à l'état solide en libérant une chaleur latente ; celle-ci va réchauffer le patient pour le maintenir dans la zone de températures de confort supérieure à 25°C, de préférence 28°C.

Un équilibre se crée ainsi au sein du dispositif et à la surface du corps du patient : la température de sa peau ne dépasse pas les 40°C, de préférence 35°C et se stabilise dans une zone de confort comprise entre 25 et 40°C, de préférence 28 et 35°C. La température corporelle du patient est donc aussi régulée, les écarts de température deviennent très limités.

D'autre part, lorsque le patient s'allonge sur le dispositif en fonctionnement, il se crée un taux élevé d'humidité sur la pièce supérieure au voisinage du patient. Ainsi, un gradient d'humidité se crée entre le dessus de la couche supérieure et le dessous de cette couche ; des molécules d'eau en phase vapeur vont donc traverser la couche supérieure, du patient vers l'intérieur du dispositif. En effet, cette pièce supérieure permet une migration des molécules d'eau en phase vapeur grâce à la nature chimique des molécules de ladite pièce supérieure. Des charges positives et négatives s'alternent sur les molécules de la pièce supérieure et les molécules de vapeur d'eau peuvent interagir avec la chaîne moléculaire, notamment de polyuréthane de la pièce supérieure par liaison hydrogène et ainsi se déplacer le long de cette chaîne moléculaire. Ainsi, elles traversent la pièce supérieure pour se retrouver à l'intérieur du dispositif. Elles sont ensuite chassées par le flux d'air. Au final, il y a diminution de l'humidité du patient.

Le flux d'air au sein de ladite chambre optimise l'efficacité du matériau PCM en absorbant une part d'énergie calorique du patient. D'autre part, l'absorption de chaleur par le matériau PCM contribue à une baisse accrue de l'humidité du fait de la réduction de la transpiration du patient. Il y a donc un effet de synergie entre les moyens spécifiques de baisse de l'humidité que sont lesdites première et deuxième pièces et flux d'air et les moyen spécifiques de régulation de la température que sont lesdites capsules de matériau PCM.

La combinaison de régulation d'humidité et de contrôle de la température avec le dispositif selon l'invention permet ainsi de réduire considérablement le risque d'escarres de décubitus conformément au but de l'invention.

Un autre avantage du dispositif selon l'invention est qu'il permet de réguler ou contrôler la température sans avoir à mettre en oeuvre un dispositif de réchauffement et/ou de refroidissement de l'air injecté et/ou de l'air à l'intérieur de ladite chambre, requérant une alimentation en énergie pour fonctionner.

De façon connue, les matériaux à changement de phase peuvent être des matériaux organiques comme des alcools gras ou des acides gras, des glycols ou de préférence des hydrocarbures paraffiniques ou des matériaux PCM inorganiques, tels que des sels hydratés. Et de façon connue, des capsules ou microcapsules de matériaux PCM de 10 µm à 5 mm présentent une paroi polymériques sphérique creuse de 1 à 50 µm d'épaisseur.

Plus particulièrement, lesdites capsules sont des microcapsules de diamètres de 10 à 500 µm, de préférence 25 à 100 µm, fixées, de préférence de manière régulièrement espacées, au sein et/ou de préférence à la surface supérieure d'un dit support perméable à l'air.

De préférence, lesdits matériaux PCM sont des hydrocarbures paraffiniques comportant au moins une chaine en C-14, dont un premier matériau PCM dont les températures de changement de phase sont situées entre 25° et 30° C, de préférence le nonadécane, et un deuxième matériau PCM dont les températures de changement de phase sont situées entre 30° et 35° C, de préférence le lycosane.

De préférence, il comprend au moins deux matériaux de type PCM dont un premier matériau PCM dont la température de cristallisation est plus basse que celle du deuxième matériau PCM et comprise entre 25° et 30°C, de préférence entre 28° et 30°C et un deuxième matériau PCM dont la température de fusion est plus élevée que la température de fusion du premier matériau PCM et comprise entre 30° et 40°C, de préférence entre 32° et 35°C.

Plus particulièrement encore, lesdites microcapsules de matériau(x) PCM sont appliquées par-dessus et/ou au sein d'un dit support constituant une couche intercalaire perméable à l'air et à la vapeur d'eau, formant un tissu dénommé tissu 3D ou constituée d'un matériau fibreux non-tissé fortement poreux, ladite couche intercalaire étant plus épaisse que lesdites première et deuxième pièces, de préférence de 5 à 50 mm d'épaisseur.

On entend ici par tissu 3D des matériaux connus tissés en trois dimensions, contrairement aux textiles courant qui sont tissés en deux dimensions (plan), ils présentent donc une certaine épaisseur, correspondant au tissage de la troisième dimension. Ces matériaux présentent de par leur constitution une forte porosité et sont donc perméables à l'air. Enfin, comme mentionné ci-dessus, ils confèrent un effet technique de protection mécanique des microcapsules de matériau PCM.

Cette couche intercalaire épaisse favorise aussi l'écartement desdites première et deuxième pièces et facilite ainsi la circulation d'air à l'intérieur de la chambre et donc sa meilleure diffusion puis une meilleure évacuation de la vapeur d'eau. Cette pièce intercalaire incluse à l'intérieur de la chambre a aussi pour effet d'éviter les ponts climatiques entre lesdites première et deuxième pièces et de permettre un meilleur passage de l'air.

Plus particulièrement, ladite pièce intercalaire épaisse est constituée d'une couche d'un matériau fibreux non tissé, de préférence de la ouate de polyester, de préférence encore maintenue en forme par un dispositif de maintien, notamment un filet et/ou un quadrillage de coutures de type matelassage.

La couche intercalaire épaisse a ainsi un effet absorbant favorisant une meilleure répartition et l'étalement de l'humidité et donc une meilleure diffusion des l'humidité dans l'intérieur de ladite chambre, l'humidité se trouvant ainsi plus rapidement évacuée par l'air injecté à l'intérieur de ladite chambre et induisant une déshumidification plus performante.

Les inventeurs ont découvert que ce mode de réalisation est particulièrement avantageux, car ledit matériau fibreux non-tissé permet que les microcapsules s'y enfoncent sans s'écraser sous le poids du corps du patient, alors qu'en absence de cette couche de matériau fibreux non-tissé intercalaire, on observe au bout d'un certain temps que lesdites microcapsules s'écrasent sous le poids du corps du patient.

Plus particulièrement encore, le dispositif comprend en outre une feuille intercalaire de matériau fibreux non-tissé, enduite au moins sur sa surface supérieure de dites microcapsules de matériau(x) PCM, lesdites microcapsules présentant un diamètre de 10 à 500 µm, de préférence de 25 à 100 µm, et étant dispersées à raison de 10⁵ à 10⁷ microcapsules/cm², de préférence de 10⁶ à 5x10⁶ microcapsules/cm², ladite feuille intercalaire étant plus mince que lesdites première et deuxième pièces, de préférence présentant une densité surfacique de 100 à 200 g/cm².

Plus particulièrement, ladite chambre intérieure comprend une dite feuille intercalaire de matériau fibreux non-tissé mince, enduite sur au moins sa face supérieure de dites microcapsules de matériau(x) PCM, ladite feuille intercalaire étant fixée par-dessus la surface supérieure de ladite couche intercalaire plus épaisse d'un matériau fibreux non-tissé, perméable à l'air et à la vapeur d'eau.

Selon d'autres caractéristiques particulières dudit dispositif:
- la perméabilité à la vapeur d'eau de ladite deuxième pièce est inférieure à celle de ladite première pièce, et
- lesdits moyens d'évacuation sont desdites perforations, disposés par rapport au(x) dit(s) orifice(s) d'injection de façon à être apte à créer une circulation de l'air entrant dans ladite chambre par ledit orifice d'injection et évacué de ladite chambre par lesdits moyens d'évacuation, de préférence lesdites perforations, dans tout le volume de ladite chambre, lorsque ladite enveloppe est gonflée par de l'air injecté continûment sous pression par ledit orifice d'injection de manière à créer une surpression dans la dite chambre, et
- ladite deuxième pièce est sensiblement étanche à l'air entre ledit orifice d'injection et lesdites zones perforées ou poreuses perméables à l'air, ces dernières étant disposées suffisamment loin du(ou des)dit(s) orifice(s) d'injection pour que sensiblement tout le volume de ladite chambre soit parcouru par de l'air circulant entre le(s)dit(s) orifice(s) d'injection et lesdites zones perforées ou poreuses.
- ladite première pièce est constituée d'un substrat poreux ou perforé non étanche à l'eau et à l'air, ledit substrat étant enduit sur au moins une face d'une couche continue de polymère, de préférence de type polyuréthane, étanche à l'eau liquide et à l'air, et présentant des propriétés de transfert moléculaire de la vapeur d'eau, et
- ladite deuxième pièce est constituée d'un tissu enduit sur au moins une de ses faces d'une couche de polymère, de préférence de type polyuréthane, présentant des propriétés de transfert moléculaire à la vapeur d'eau, de préférence sur la face tournée du côté de l'intérieur de ladite chambre.

Pour ladite première pièce, un substrat poreux ou perforé tel qu'un tissu résultant du tissage de fibres ou de fils présente des porosités ou perforations ne faisant pas barrière au passage de la vapeur d'eau transférée à travers la couche de polymère de type polyuréthane.

Des polymères et matériaux textiles de ce type à transfert de vapeur d'eau sont connus de l'homme de l'art et disponibles dans le commerce et utilisés notamment dans l'industrie du vêtement pour leur qualité de respiration du corps et élimination/régulation de la transpiration.

Cette propriété de transfert moléculaire de la vapeur d'eau desdits polymères résulte de l'affinité moléculaire induisant une attraction des molécules d'eau sur les chaînes moléculaires du polymère, notamment du polyuréthane, comprenant des groupes hydrophiles, lesdites molécules d'eau pouvant ainsi cheminer le long de la chaîne polymère et traverser la couche de dit polymère.

Avantageusement, ladite deuxième pièce est constituée d'un tissu enduit sur au moins une de ses faces d'une couche de polymère perméable à la vapeur d'eau, c'est-à-dire présentant des propriétés de transfert moléculaire de la vapeur d'eau, de préférence sur la face tournée du côté de l'intérieur de ladite chambre.

Dans un mode de réalisation préféré, lesdites première et deuxième pièces sont reliées entre elles sur leurs bords périphériques par soudage, directement l'une avec l'autre ou par l'intermédiaire d'une bande de liaison revêtue d'une couche de polymère, les différentes couches de polymère desdites première et deuxième pièces et de ladite bande, le cas échéant, étant soudables entre elles par thermo soudage ou soudage par irradiation, de préférence irradiation haute fréquence.

Plus particulièrement, les différentes couches de polymère sont de même nature chimique ou de nature chimique aptes à les rendre soudables entre elles, ce qui est le cas des polymères de type polyuréthane.

Les deux dites pièces peuvent être revêtues de polymère sur leurs deux faces, et être scellées l'une à l'autre directement. Les deux pièces sont, de préférence, pour des raisons économiques, revêtues de polymère sur une face seulement. Et, si l'une au moins des faces internes des deux pièces n'est pas revêtue de polymère, notamment si la première pièce est revêtue de polymère sur sa face externe et la deuxième pièce sur sa face interne, le bord de l'une des deux pièces peut être replié sur lui-même de manière à présenter sa face revêtue de polymère vers l'intérieur de ladite chambre.

Dans un mode de réalisation, on peut également avantageusement mettre en oeuvre une bande de liaison périphérique éventuellement repliée sur elle-même, assurant la liaison par soudage entre les deux dites première et deuxième pièces, ladite bande étant elle-même revêtue sur une face au moins d'une couche de polymère soudable avec les couches de polymère des deux dites première et deuxième pièces, la couche de polymère de ladite bande étant de même nature imperméable à l'eau liquide, à l'air et à la vapeur d'eau que celle de ladite deuxième pièce.

De façon avantageuse, lesdits tissus constitutifs desdites première et deuxième pièces sont des tissus extensibles dans les deux directions longitudinale et transversale. Plus particulièrement, cette propriété permet de favoriser une meilleure répartition du poids du corps, c'est-à-dire sur sa plus grande surface, et que le tissu épouse la forme du corps et ne génère pas des points localisés de compression excessive du corps pouvant générer des blocages de vascularisation et des pathologies qui en résultent.

Avantageusement, lesdites perforations d'évacuation d'air sont disposées sur la périphérie de ladite deuxième pièce. Ces zones périphériques ne coïncident pas avec la zone centrale de l'enveloppe sur laquelle le corps est normalement en appui, mais entourent celle-ci, facilitant ainsi l'évacuation de l'air par lesdites perforations.

Dans un mode de réalisation préféré, ladite deuxième pièce est de forme sensiblement rectangulaire et le dispositif comprend un unique orifice d'injection à proximité du milieu d'un bord longitudinal de ladite deuxième pièce, lesdites perforations d'évacuation d'air étant disposées à proximité des bords latéraux et du bord longitudinal opposé à celui de l'orifice d'injection.

Ainsi, l'orifice d'injection est disposé par rapport aux dites perforations d'évacuation de manière à bien faire circuler l'air partout dans la chambre interne de l'enveloppe et évacuer à l'extérieur le plus rapidement possible l'humidité transférée depuis le voisinage ou la surface du corps vers l'intérieur de ladite chambre.

La présente invention fournit également un procédé de régulation de l'humidité et de la température à la surface d'un élément de support de type matelas ou coussin et au voisinage et contact du corps d'un individu y reposant, à l'aide d'un dispositif selon l'invention, **caractérisé en ce qu'**on réalise les étapes dans lesquelles :
1) on dispose à plat ladite enveloppe entre ledit élément de support et ledit corps d'un individu, de telle sorte que ladite première pièce soit tournée du côté du corps de l'individu et ladite deuxième pièce soit tournée du côté dudit élément de support, et
2) on injecte de l'air sous pression dans ladite chambre par ledit orifice d'injection à une pression et à un débit tels que ladite enveloppe reste gonflée en surpression en dépit de l'évacuation de l'air par lesdits moyens d'évacuation, de préférence desdites perforations et de l'appui du corps sur ladite enveloppe, ladite surpression de l'air à l'intérieur de l'enveloppe par rapport à l'extérieur étant suffisante pour permettre une circulation de l'air dans tout le volume de la dite chambre.

On comprend que l'on injecte l'air à une pression et à un débit tels que l'air à l'intérieur de la chambre soit en surpression par rapport à l'air à l'extérieur de la chambre.

De par les propriétés de perméabilité à la vapeur d'eau de ladite première pièce, un transfert de vapeur d'eau s'opère à travers ladite première pièce lorsqu'un gradient d'humidité relative existe entre la surface externe de ladite première pièce de l'enveloppe et l'intérieur de ladite chambre, notamment en cas de macération d'un liquide ou de sudation du corps ou à proximité du corps entre le corps et ladite enveloppe, ce qui s'accompagne d'une déshumidification de la surface de ladite première pièce. Lorsque le taux d'hygrométrie est identique des deux côtés de ladite première pièce, c'est-à-dire entre l'extérieur et l'intérieur de ladite enveloppe, il n'y a plus de gradient d'humidité relative et le transfert de vapeur d'eau s'interrompt automatiquement. Mais, la circulation d'air à l'intérieur de l'enveloppe permet d'entraîner la vapeur d'eau, de l'évacuer à l'extérieur par ladite deuxième pièce. Cette circulation favorise donc une diminution de l'hygrométrie à l'intérieur de l'enveloppe et entretient le transfert de vapeur d'eau depuis l'extérieur de l'enveloppe vers l'intérieur de l'enveloppe le cas échéant, tant que l'humidité à l'extérieur de celle-ci est supérieure à l'humidité relative de l'air à l'intérieur de l'enveloppe et donc à l'humidité de l'air ambiant injecté. Le fait que l'humidité à l'extérieur de l'enveloppe au niveau de la surface de l'enveloppe soit diminuée jusqu'à atteindre la valeur d'humidité relative de l'air ambiant injecté, et que le transfert de vapeur d'eau s'interrompe automatiquement à ce moment, permet d'éviter une déshydratation excessive des tissus de la peau dudit corps. En outre, du fait des propriétés de perméabilité à la vapeur d'eau de ladite deuxième pièce, la vapeur transférée à l'intérieur de ladite chambre peut s'évacuer vers l'extérieur en dépit de la circulation d'air, ce qui permet de maintenir et favoriser l'établissement d'un gradient d'humidité relative élevé entre l'extérieur de ladite première pièce et l'intérieur de ladite chambre.

Enfin, le fait que ladite deuxième pièce est perméable à la vapeur d'eau, et que l'air est évacué exclusivement à travers ladite deuxième pièce, permet de fournir un procédé de déshumidification exclusivement par transfert de vapeur d'eau par migration moléculaire à travers le dispositif et ce, avec un taux de transfert de vapeur d'eau et donc de déshumidification plus élevé que dans la technique antérieure, sans risque d'humidification excessive au-dessous du dispositif entre ladite deuxième pièce et le matelas, sans risque de contamination par pénétration de contamination ou de liquide provenant de la surface externe supérieure de ladite première pièce puisque celle-ci est non perforée, et enfin, sans requérir de traitement d'hydratation concomitant du patient comme c'est le cas lorsque de l'air est évacué de la couche supérieure en direction ou à proximité du patient.

Par ailleurs, du fait que selon la présente invention, l'air est évacué par ladite deuxième pièce ou couche inférieure, la vapeur d'eau transférée en sous-face de ladite deuxième pièce ou couche inférieure ne s'accumule pas entre celle-ci et le matelas sur lequel elle est disposée, et est évaporé par l'air évacué qui y est ainsi injecté.

Comme explicité précédemment, l'ajout de la couche de PCM permet de stabiliser la température de la peau (et donc du corps) et par conséquent de limiter la transpiration. D'autre part, on constate un effet synergique, dont le mécanisme n'est pas totalement élucidé, en ce que la circulation d'air du dispositif augmente de façon surprenante l'efficacité des microcapsules de PCM, d'où un rafraîchissement plus efficace qu'en l'absence de circulation d'air.

Dans un mode préféré de réalisation de la présente invention, la perméabilité à la vapeur d'eau de ladite deuxième pièce est inférieure à celle de ladite première pièce.

Cette perméabilité à la vapeur d'eau limitée de ladite deuxième pièce permet d'éviter l'accumulation d'humidité entre ladite deuxième pièce et ledit élément de support et permet l'évaporation de cette humidité par la seule circulation d'air provenant desdits moyens d'évacuation d'air en l'absence d'injection d'air additionnel entre ladite deuxième pièce et ledit élément de support.

Avantageusement, ladite deuxième pièce est sensiblement étanche à l'air entre ledit orifice d'injection et lesdites zones perforées ou poreuses perméables à l'air, ces dernières étant disposées suffisamment loin dudit orifice d'injection pour que sensiblement tout le volume de ladite chambre soit parcouru par de l'air circulant entre ledit orifice d'injection et lesdites zones perforées ou poreuses.

Avantageusement, selon l'invention, le rapport entre les sections cumulées des perforations d'évacuation et de la section des orifices d'injection est choisi de manière à obtenir un compromis entre d'une part la recherche d'un débit de flux d'air circulant à travers la chambre élevé et d'autre part une surpression suffisante à l'intérieur de la chambre. En effet, la surpression doit être suffisante pour garantir que l'air injecté circule dans tout le volume de la chambre, c'est-à-dire que l'air soit uniformément réparti dans tout le volume de la chambre. Autrement il y a un risque que l'air injecté ne se borne à circuler à l'intérieur de ladite chambre seulement entre le(s)dit(s) orifice(s) d'injection et celles des dites zones localisées perforées ou poreuses correspondant au passage de perte de charge minimale.

Toutefois, on comprend que la surpression doit être limitée de façon à ne pas déstabiliser le corps de l'individu en appui sur ladite enveloppe.

En pratique une surpression d'au moins 500Pa permet d'obtenir une circulation d'air homogène dans toutes les directions et notamment dans la zone au dessous ou en vis-à-vis du corps du patient.

Par ailleurs, la limite supérieure de débit d'air circulant dans la chambre est lié au taux maximum de vide, c'est-à-dire à la section cumulée des orifices que peut tolérer le matériau de la dite deuxième pièce d'un point de vue de sa résistance mécanique. Ce taux ne devrait pas dépasser 10% en général. En outre, il y a lieu de prendre en compte que l'effet positif de l'augmentation du débit d'air sur la performance de déshumidification est limité par la capacité de transfert de la vapeur d'eau des dites première et deuxième pièce couche. Au-delà d'un certain débit d'air les performances de déshumidification ne sont plus améliorées.

En pratique un débit de 20 à 501/min apporte des performances de déshumidification suffisante compte tenu du temps de migration des molécules d'eau pour le transfert de la vapeur d'eau à travers les couches de polymères mises en oeuvre comme décrit ci-après.

Plus particulièrement, pour obtenir une surpression de 500 à 1000Pa avec un débit de circulation d'air de 20 à 50 l/min., le rapport de la somme des sections des perforations ou pores desdites zones perforées ou respectivement poreuses d'évacuation de l'air par rapport à la somme des sections des orifices d'injection est d'au moins 2, de préférence de 2 à 4.

L'évaporation de l'eau en surface du côté du patient s'accompagne d'une diminution légère de la température qui favorise une diminution de la sudation le cas échéant et compense l'augmentation de température résultant de la compression de l'air injecté.

Plus particulièrement, le transfert de vapeur d'eau de ladite première pièce est d'au moins 750, de préférence 750 à 2000 g. d'eau/m²/24h, de préférence encore environ 1000 g.d'eau/m²/24h et le transfert de vapeur d'eau de la deuxième pièce est inférieur à 500 g.d'eau/m²/24h, de préférence de 300 à 500 g.d'eau/m²/24h.

Dans un mode préféré de réalisation du procédé selon l'invention, on établit une surpression de l'air à l'intérieur de ladite chambre par rapport à l'extérieur de la chambre, suffisante pour que l'air circule dans tout le volume de la dite chambre et plus particulièrement on établit une surpression de 500 à 1000 Pa, de préférence environ 750 Pa, avec un débit d'air circulant dans ladite chambre est d'au moins 20 I/minute, de préférence 30 à 50 l/minute.

Avantageusement, le dispositif selon l'invention comporte en outre un dispositif d'injection d'air comprimé alimentant en air ladite enveloppe par un dit orifice d'injection.

Avantageusement encore, dans le procédé selon l'invention, on injecte en outre de l'air entre ladite deuxième pièce et ledit élément de support, de préférence à partir d'un même dispositif d'injection d'air comprimé alimentant ladite enveloppe par ledit orifice d'injection.

Dans un mode préféré de réalisation, ledit élément de support est constitué d'au moins un compartiment gonflable, rempli d'air, de préférence relié à un même dispositif d'injection d'air que celui alimentant ladite enveloppe.

L'air emplissant ladite chambre peut être ainsi dérivé de la même source d'air que celle permettant de gonfler le matelas par l'intermédiaire d'un dispositif orientant l'air de manière sélective, par exemple une électrovanne faisant office de dispositif d'aiguillage d'une source d'air unique. Ladite électrovanne peut être partie intégrante d'un module coopérant avec le dispositif selon l'invention.

Des installations d'injection d'air dans un matelas gonflable ont été décrites notamment dans les brevets de la demanderesse EP 676 158, FR 2 751 743, FR 2 757 377, FR 2 757 378, FR 2 758 259, FR 2 760 967.

Avantageusement encore, le dispositif selon l'invention peut comprendre en outre un système de commande à distance, notamment par télécommande, dudit dispositif d'injection d'air comprimé.

De préférence encore, le dispositif de l'invention est intégré à une housse de protection d'un dit élément de support de type matelas ou coussin, au moins au niveau de la partie de la housse destinée à recouvrir la partie de la face de l'élément support sur laquelle une partie au moins du corps d'un individu est destinée à reposer.

D'autres avantages et caractéristiques de la présente invention apparaîtront à la lumière de la description détaillée qui va suivre et aux figures 1 à 6 dans lesquelles :
- La figure 1 représente une vue schématique en coupe longitudinale d'un matelas 13 gonflé à air recouvert d'une housse de protection incorporant le dispositif de régulation 1 selon la présente invention et sur lequel repose un patient 14,
- La figure 2 représente une vue schématique des 4 pièces composant un dispositif de régulation 1 selon l'invention,
- La figure 3 représente en coupe transversale de façon schématique un mode de soudure de la pièce inférieure sur la pièce supérieure par l'intermédiaire d'un bandeau 6 formant rebord latéral 6₁ d'une housse se terminant par un dispositif de fermeture/ouverture à glissière 6₂ et formant aussi un rabat 6₃ de protection d'un dispositif de fermeture/ouverture 6₂,
- La figure 4 est une représentation schématique d'un tissu 2 incorporant des microcapsules régulièrement espacées sur toute la surface du tissu 2, ledit tissu 2 étant cousu par des coutures de matelas et un surfilage périphérique 2b par-dessus une couche poreuse à l'air plus épaisse 3,
- La figure 5 représente des courbes de mesures de la température de la peau en °C sous le patient allongé par-dessus un dispositif 1 selon l'invention par-dessus un matelas 13, en fonction du temps (e) en secondes,
   la courbe A représentant un mode de réalisation dans lequel le dispositif 1 comprend un tissu PCM 3 cousu par-dessus une couche de ouate de polyester 2, le dispositif de régulation 1 étant traversé par un flux d'air dans la chambre 1₃,
   la courbe B correspondant au même mode de réalisation avec tissu PCM 3 et flux d'air, mais sans couche de ouate de cellulose 2 dans la chambre 1₃.
   la courbe C représentant un même mode de réalisation que la courbe B mais sans flux d'air, et
   la courbe D représentant le même mode de réalisation que la courbe A avec flux d'air mais sans tissu PCM 3.

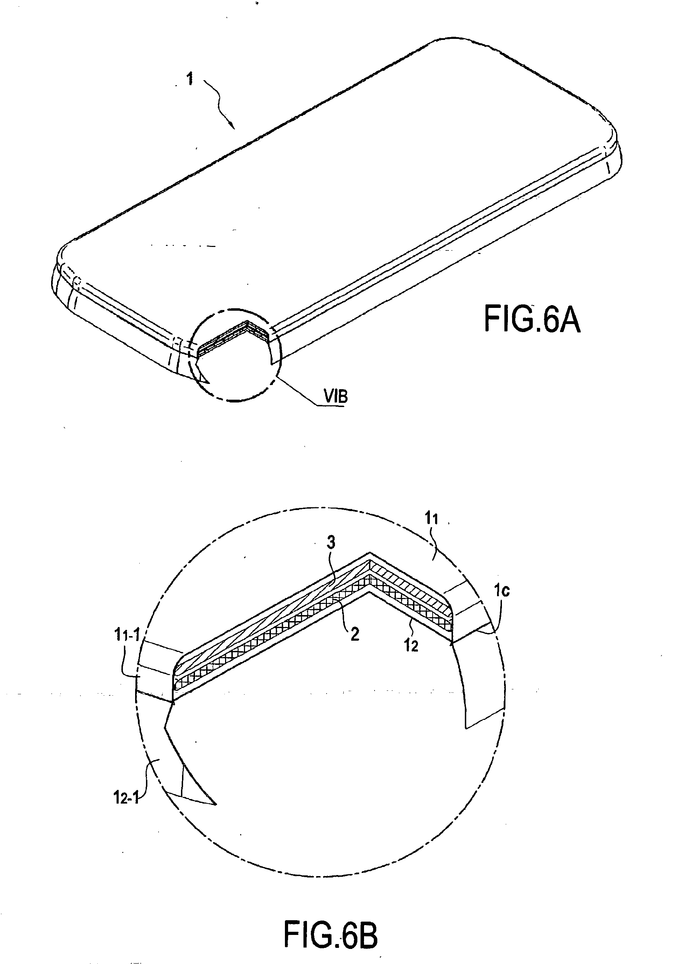

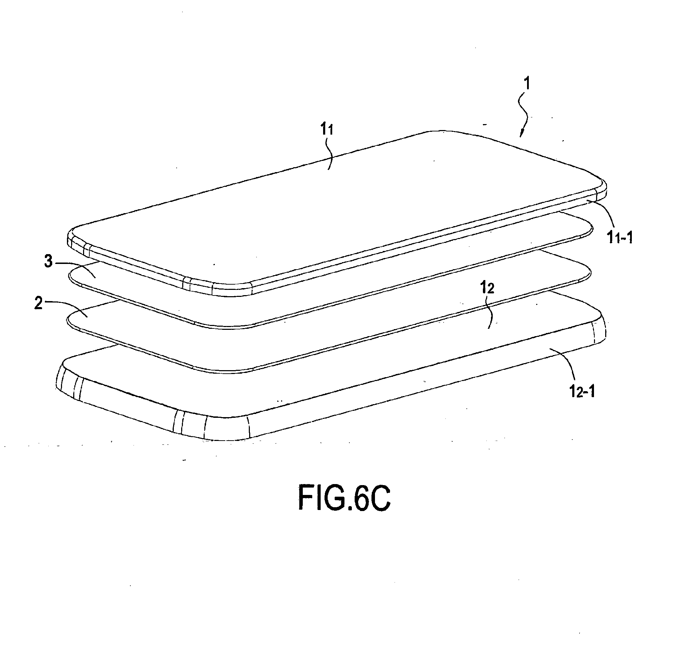

- Les figures 6A, 6B et 6C sont des vues en perspective (fig. 6A), en détail (fig. 6B) et en éclaté (fig. 6C) d'une seconde variante de réalisation d'un dispositif 1 selon l'invention, pouvant être appliqué comportant une pièce inférieure 1₂, doté d'une jupe périphérique 1₂-1, apte à recouvrir les bords latéraux d'un matelas 13 sur lequel on applique le dispositif 1 tel quel, la pièce supérieure 1₁, comportant également une jupe périphérique 1₁-1 thermo-soudée sur le bord périphérique supérieur de la pièce inférieure 1₂.

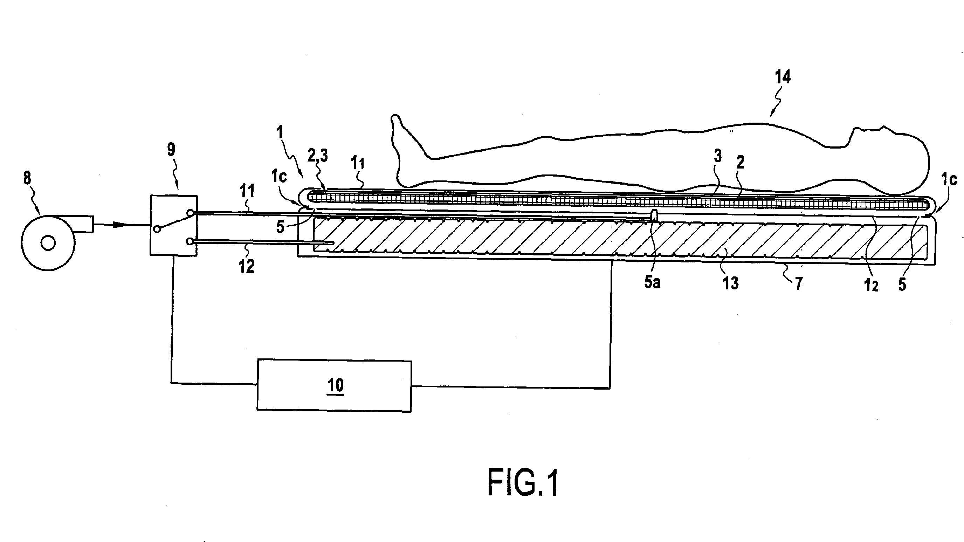

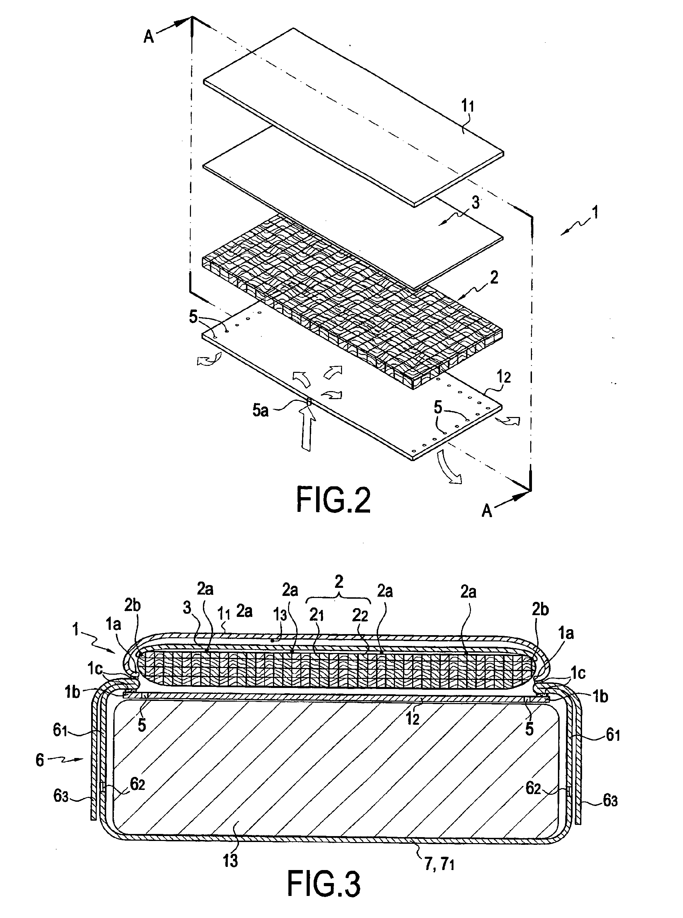

Sur les figures 1 à 3, on a représenté un dispositif de régulation selon l'invention comprenant une enveloppe 1 formée à partir de 2 pièces : une pièce supérieure ou dite première pièce 1₁ et une pièce inférieure ou dite deuxième pièce 1₂, dont les bords périphériques 1a et 1b sont soudés 1c entre eux par l'intermédiaire d'une bande de liaison 6 repliée sur elle-même, les lignes de soudure 1c suivant sensiblement un contour rectangulaire.

L'enveloppe 1 du dispositif de régulation selon la présente invention est intégrée à une housse de protection 7 recouvrant un matelas à air 13. Plus particulièrement, l'enveloppe 1 forme la face supérieure de la housse de protection, et une partie de la bande de liaison 6 forme des rebords latéraux 6₁ de la housse de protection recouvrant en partie les flancs du matelas 13, ladite deuxième pièce inférieure ou dite deuxième pièce 1₂ étant appliquée directement sur le matelas 13.

Lesdites pièces supérieure et inférieure sont donc de forme sensiblement rectangulaire correspondant sensiblement aux dimensions du matelas. La housse comporte un dispositif de fermeture/ouverture à glissière à dents 6₂ périphérique le long des côtés ou rebords latéraux de la housse recouvrant la tranche du matelas, ledit dispositif de fermeture/ouverture 6₂ permettant la séparation d'une partie inférieure 7₁ de housse et des rebords latéraux 6₁ et le retrait du matelas de la housse.

La pièce supérieure ou première pièce 1₁ est constituée d'un tissu de fils de polyester revêtus sur sa face supérieure externe d'un polymère de type polyuréthane présentant des propriétés de transfert à la vapeur d'eau.

Plus précisément, ces polymères de type polyuréthane sont constitués de chaînes moléculaires de polyuréthane contenant des groupes hydrophiles ester permettant un transfert de vapeur d'eau par migration moléculaire des molécules d'eau par le biais d'interaction physico-chimiques avec lesdits groupes hydrophiles ester desdites chaînes moléculaires.

De tels tissus revêtus sur une face de polyuréthane à propriétés de transfert de vapeur d'eau sont commercialisés sous la marque Dartex^{®}, notamment sous la référence P510, présentant des propriétés de transfert de vapeur d'eau d'environ 1000 g. d'eau/m²/24h (quantité d'eau transférable à travers le tissu enduit), et comprenant une composition de 66% de polyester et 34% de polyuréthane, et un grammage de 130 g/m².

La pièce inférieure ou deuxième pièce 1₂ est constituée d'un tissu de nylon à base de polyamide enduit sur une face d'une couche de polyuréthane perméable à la vapeur d'eau, commercialisé par la société Dartex^{®}, notamment sous la référence P280 avec un taux de transfert de vapeur d'eau d'environ 350 g.d'eau/m²/24h, une composition de 47% de polyamide et 53% de polyuréthane, et un grammage de 179 g/m².

L'enveloppe constituée par les deux pièces inférieure et supérieure délimite une chambre interne 1₃. A l'intérieur de la chambre, on insère une première pièce intercalaire 2 occupant sensiblement tout le volume de la chambre de forme sensiblement rectangulaire, constituée d'une couche d'un matériau non tissé 2₁ de 5 à 10 mm d'épaisseur, constituant une couette à base de ouate de cellulose de 150 à 200 g/m² constituant un matériau absorbant perméable à l'air et à l'eau. On peut utiliser notamment un tissu 3D de la société AMES EUROPE de référence Pro643/3. Cette couche intercalaire présente la double propriété de répartir et diffuser de manière homogénéisée la vapeur d'eau transférée à l'intérieur de la chambre depuis la surface externe de la pièce supérieure, et constituer un espaceur entre les pièces inférieure et supérieure pour éviter le contact entre les deux pièces, et, en outre, assurer une certaine protection mécanique des microcapsules de PCM décrites ci-après.

La première pièce intercalaire de matériau non tissé 2 est recouverte d'un filet synthétique 2₁ du type tulle à fibres de polyester. Une couture périphérique solidarise la couche de la pièce intercalaire avec ledit filet, de préférence des coutures en quadrillage longitudinal et latéral permettent de stabiliser la forme de la première pièce intercalaire.

Une deuxième pièce intercalaire 3 constituée d'un tissu, dénommé ci-après « tissu PCM », est cousue sur la face supérieure de la première pièce intercalaire. Le tissu PCM 3 est cousu en quadrillage à la surface du tissu 3D 2, de façon à ce qu'il ne fasse pas de plis et qu'il ne bouge pas lorsqu'on est amené à emballer par exemple, à emballer, plier et/ou enrouler l'ensemble du dispositif 1 pour le transporter. Le tissu PCM 3 est constitué d'une couche de polyester non-tissée, revêtu d'un enduit acrylique contenant des microcapsules 4 de matériau PCM. Lesdites microcapsules 4 sont de diamètre d'environ 40 µm. Lesdites microcapsules sont constituées d'une paroi membranaire sphérique d'environ 2 µm d'épaisseur, incluant le matériau PCM. Le tissu PCM 3 comprend plus particulièrement environ 3.000.000 de microcapsules/cm². Le matériau PCM inclus dans les microcapsules est un mélange d'hydrocarbures paraffiniques, à savoir le nonadécane et le lycosane dont les températures de fusion respectives sont d'environ 28 et 33°C.

Ce tissu PCM 3 est perméable à l'air et à l'eau et présente une densité surfacique (y compris avec les microcapsules de PCM) de 140 gr/m².

Un tissu PCM 3 de ce type est commercialisé par la société Outlast Technology (USA) sous la référence commerciale 233 Outlast 901 greeley. Des tissus PCM de ce type sont également commercialisés par la société Schoeller Textil (Suisse).

Dans un mode de réalisation, on injecte de l'air de manière à créer une surpression d'environ 750 Pa dans l'enveloppe 1 par rapport à l'air extérieur en établissant un débit de flux d'air entrant et de flux d'air sortant équilibré de 25 à 35 I/minute. Pour ce faire, la pièce inférieure 1₂ comprend un orifice d'injection d'air 5a au centre d'environ 9,5mm et à proximité d'un bord longitudinal de la pièce inférieure 1₂, ledit orifice d'injection d'air étant constitué d'un connecteur plastique soudé. Des perforations d'évacuation d'air de diamètre d'environ 3 mm sont espacées de 10 à 20 cm régulièrement le long des trois autres bords de la pièce inférieure 1₂, c'est-à-dire les deux bords transversaux et le bord longitudinal opposé à celui de l'orifice d'injection. Pour un lit d'environ 2m de long sur 90 cm de large, on a ainsi réalisé 48 perforations. Les sections cumulées des perforations d'évacuation 5 représentent ainsi environ le double de la section de l'orifice d'injection 4. Ainsi, on compense les pertes de charge liées aux restrictions au passage d'air et maintient un équilibre entre les débits entrant et sortant de flux d'air avec ce débit de 25 à 35 I/min.et cette surpression de 750 Pa environ de l'air à l'intérieur de la chambre par rapport à l'extérieur.

L'orifice d'injection d'air 5a est alimenté par un compresseur 8 qui permet également d'alimenter le matelas à air 13 par l'intermédiaire d'une électrovanne 9 qui sert d'aiguillage commandé par un dispositif d'asservissement 10, soit pour l'alimentation en air 12 du matelas 13, soit par l'alimentation en air 11 de l'orifice d'injection d'air à l'intérieur de ladite chambre, en fonction des mesures d'un capteur de pression du gonflage de matelas notamment.

Le dispositif de régulation selon l'invention permet d'assécher ainsi 500ml d'eau répandus uniformément sur un drap de coton aux dimensions du matelas, soit environ 2m² en 3h30 lorsque ledit matelas est recouvert d'un mannequin simulant le corps d'un patient en présence desdites première et deuxième pièces intercalaires 2 et 3 et en 6h en l'absence de ladite première pièce intercalaire 2, les essais ayant été réalisés dans une atmosphère ambiante de 40% d'humidité relative environ et à température ambiante d'environ 25°C.

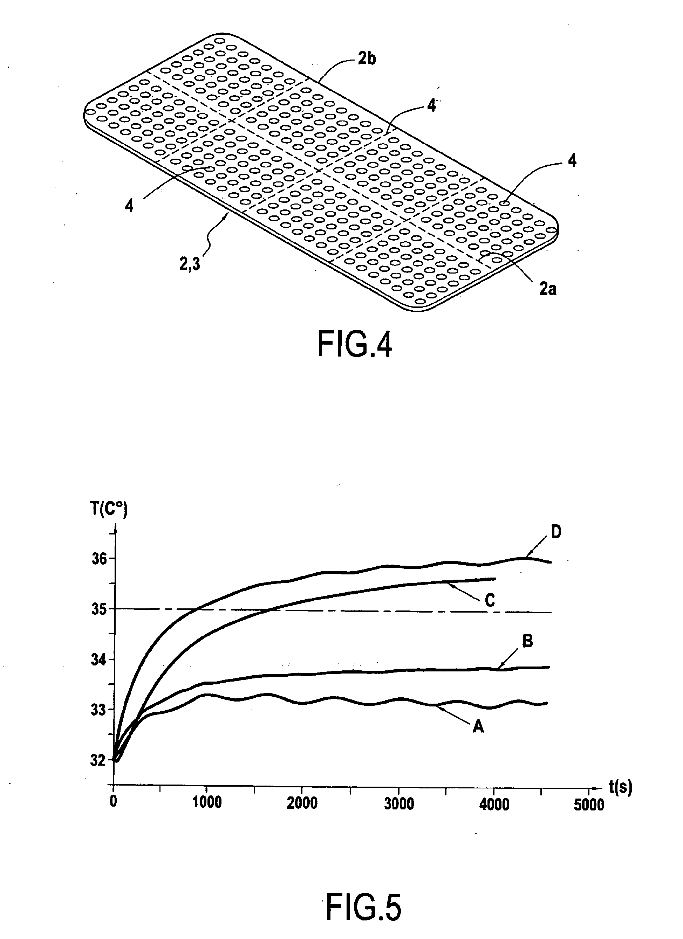

Sur la figure 5, on a représenté plusieurs courbes de mesure de la température de la peau sous le patient 14 en contact sur un matelas 13 équipé d'un dispositif de régulation de l'humidité de la température 1 dans les conditions suivantes :
- courbe A = un dispositif 1 comprenant un tissu PCM 3 combiné à une couche épaisse poreuse de ouate de cellulose 2 avec un flux d'air crée tels que décrits ci-dessus,
- courbe B = un même dispositif 1 avec un même tissu PCM 3, mais en l'absence de couche épaisse 2 avec un même flux d'air,
- courbe C = même dispositif 1 que la courbe B, mais sans flux d'air, et
- courbe D = même dispositif 1 que la courbe A, mais sans tissu PCM 3 et avec un même flux d'air.

On voit que, seules les courbes A et B restent en-dessous de 35°C dans le temps, alors que pour les courbes C et D la température de la peau dépasse 35°C au bout de quelques minutes.

La comparaison des courbes A à D démontre que :
- la mise en oeuvre d'un tissu PCM 3 ne peut effectivement contrôler la température qu'en combinaison avec une circulation d'air et donc des moyens permettant cette circulation d'air dans lesdites couches inférieures et supérieures notamment, et
- à plus forte raison, la limitation plus importante de température (courbe A) est obtenue dans les conditions favorisant au mieux la circulation d'air par la présence d'une couche épaisse poreuse 2, et
- en l'absence de tissu PCM avec flux d'air (courbe D) ou en présence de tissu PCM sans flux d'air (courbe C), il n'est pas possible de maintenir la température en-dessous de 35°C.

Pour réaliser les courbes A à D de la figure 5, la température ambiante était d'environ 25°C. Des essais comparatifs ont permis de montrer que le dispositif de régulation selon l'invention 1 permet d'assécher 500 ml d'eau répandus uniformément sur un drap de coton tel que décrit ci-dessus en environ sept heures, lorsque ledit matelas est recouvert du corps d'un patient et que le dispositif 1 comprend uniquement une deuxième pièce intercalaire 2, et en dix heures lorsque ladite deuxième pièce intercalaire 2 est combinée à un tissu PCM 3. Des essais ont été réalisés dans les mêmes conditions d'atmosphère ambiante d'humidité et de température ambiante que décrit ci-dessus. On en déduit que la plus grande rapidité de déshumidification avec un tissu PCM 3 provient vraisemblablement de la réduction de la transpiration du corps du patient.

On voit que, en l'absence de tissu PCM 3, le dispositif 1 apporte un contrôle de l'humidité mais présente une limite d'efficacité thermique, car la courbe mesurée de température de la peau du patient alité sur le dispositif augmente avec le temps et dépasse la température critique de confort de 35°C en l'absence de tissu PCM 3. Dès lors, au-delà de cette température, le patient alité se trouve dans un état à risque de l'apparition d'escarres de décubitus dans des zones du corps à risque, telles que la zone du sacrum et celles des talons par exemple. Le tissu PCM mis en oeuvre sur une couche épaisse 2 en combinaison avec un flux d'air permet que la température de la peau reste dans la zone de confort, se situant entre 28 et 35°C.

Ceci résulte du fait que les matériaux PCM comprennent un mélange de deux matériaux PCM suivants :
- un premier matériau PCM dont la température de cristallisation la plus basse est supérieure à 28°C, de manière à ce qu'une partie desdites microcapsules de dit premier matériau PCM soit à l'état solide à la température de 28°C et que si la température au contact du patient et du dispositif 1 selon l'invention descend en-dessous de 28°C, ledit premier matériau PCM se solidifie et dégage de la chaleur, tandis que
- le deuxième matériau PCM présente une température de fusion la plus élevée et inférieure à 35°C, à savoir environ 33°C de sorte que si la température au contact du patient et du dispositif 1 s'élève au-dessus de 35°C, ledit second matériau PCM fond et absorbe de la chaleur.

D'autre part, les courbes comparatives (B) et (D) sur la figure 5 montrent qu'un même tissu PCM 3 sans première pièce intercalaire de ouate de polyester 2 (B) permet encore de maintenir la température de la peau sous le patient inférieure à 35°C, sous réserve d'être combiné à un dit flux d'air. En effet, en l'absence de flux d'air et en présence de tissu PCM 3 mais sans ouate de polyester 2, la courbe (D) dépasse les 35°C.

Les changements de phase des matériaux PCM nécessitent de l'énergie appelée « chaleur latente ». On voit que la combinaison de ces deux matériaux PCM a pour effet de limiter les écarts de température et tendre à les maintenir dans une fourchette de température restreinte correspondant à une température de confort.

La combinaison du matériau tissu PCM 3 avec un flux d'air (courbe A) et davantage encore avec en outre une couche poreuse épaisse 2 (courbe B), augmente l'effet de stabilisation de la température en-dessous de 35°C par rapport à un même dispositif 1 sans air et avec tissu PCM 3 (courbe C) et davantage encore par rapport à un même dispositif 1 sans tissu PCM 3 et avec air (courbe D).

La présence d'un tissu PCM 3 en combinaison avec un flux d'air, ledit flux d'air étant favorisé en présence d'une couche épaisse poreuse, permet de réguler la température corporelle du patient dans une fourchette de température d'écart réduit entre 28 et 34,5°C. Mais, de surcroît, la combinaison de cette combinaison d'un tissu PCM 3 avec un flux d'air et de plus forte raison en combinaison avec une couche poreuse épaisse 2, permet de diminuer d'avantage encore le taux d'humidité sur la couche supérieure au voisinage du patient, du fait que le contrôle de la température permet d'éviter, ou en tous les cas, de réduire fortement la transpiration du patient.

Sur la figure 1, la liaison par soudage des pièces inférieure 1₂ et supérieure 1₁ est réalisée par l'intermédiaire de bandes de liaison 6. Plus précisément, une bande de liaison 6 constituée d'un tissu revêtu sur une de ses faces d'une couche de polymère polyuréthane et présentant les mêmes propriétés d'imperméabilité à l'air, à l'eau et à la vapeur d'eau et repliée sur elle-même pour être soudée à la fois sur le bord périphériques la replié sur lui-même de la pièce supérieure 1₁ et sur le bord périphérique 1b de la pièce inférieure 1₂.

La bande de liaison 6 comprend donc 2 parties repliées l'une sur l'autre comprenant depuis la soudure 1c avec la pièce inférieure 1₂ une partie formant rebord latéral 6₁ du matelas 13 et se terminant par un dispositif de fermeture à glissière à dents 6₂ permettant par ouverture de sortir le matelas de la housse 7. Le rebord latéral 6₁ vient recouvrir les flancs du matelas 13. L'autre partie de la bande s'étendant depuis la soudure 1c avec la pièce supérieure 1₁ constitue un rabat 6₃ venant recouvrir ledit rebord latéral 6₁ et le dispositif de fermeture à glissière 6₂ ainsi protégé.

Avantageusement, ladite enveloppe comprend un autre dispositif d'ouverture/fermeture à glissière, non représenté, étanche à l'air et à l'eau du type des dispositifs utilisés pour sceller de manière réversible des sachets contenant des aliments permettant ainsi d'ouvrir l'enveloppe et de retirer lesdites première et deuxième pièces intercalaires 2 et 3 pour la nettoyer régulièrement.

Sur les figures 6A, 6B, 6C on a représenté une deuxième variante de réalisation simplifiée, dans lesquelles le dispositif de régulation 1 selon l'invention fait office de housse de protection d'un matelas en appliquant directement la sous-face de la deuxième pièce 1₂ par-dessus la face supérieure du matelas 13, la jupe périphérique 1₂-1 de la première pièce 1₂ venant protéger les bords latéraux verticaux du matelas 13.

## Revendications

1. Dispositif de régulation de l'humidité et de la température (1) à la surface d'un élément de support (13) du type matelas ou coussin et au voisinage et contact du corps (14) d'un individu y reposant, comprenant une enveloppe formée par au moins deux pièces (1₁, 1₂) de préférence reliées entre elles au niveau de leurs bords périphériques (1a, 1b), de préférence encore scellés par soudage (1c), définissant une chambre intérieure (1₃), lesdites deux pièces consistant dans une première pièce (1₁) destinée à être disposée du côté dudit corps de l'individu, et une deuxième pièce (1₂) destinée à être disposée du côté dudit élément de support (13) de type matelas ou coussin, ladite première pièce étant constituée d'un matériau formant une barrière imperméable à l'air et à l'eau liquide et perméable à la vapeur d'eau, ladite deuxième pièce étant constituée d'un matériau perméable à la vapeur d'eau, ladite deuxième pièce comprenant au moins un orifice (5a) d'injection d'air et des moyens d'évacuation d'air comprenant des zones perforées ou poreuses perméables à l'air, de préférence des perforations (5), **caractérisé en ce que** ladite chambre intérieure (1₃) comprend des capsules (4) de matériau(x) à changement de phase (PCM) dont la température de cristallisation la plus basse est supérieure à 25°C, de préférence supérieure à 28°C, et la température de fusion la plus élevée est inférieure à 40°C, de préférence inférieure à 35°C, lesdites capsules étant intégrées au sein et/ou disposées en surface d'un support, de préférence un support constitué d'un matériau fibreux tissé ou non-tissé, perméable à l'air (2,3).

2. Dispositif selon la revendication 1, **caractérisé en ce que** lesdites capsules sont des microcapsules de diamètres de 10 à 500 µm, de préférence 25 à 100 µm, fixées, de préférence de manière régulièrement espacées, au sein et/ou de préférence à la surface supérieure d'un dit support perméable à l'air (2,3).

3. Dispositif selon la revendication 1 ou 2, **caractérisé en ce qu'**il comprend au moins deux matériaux de type PCM dont un premier matériau PCM dont la température de cristallisation est plus basse que celle du deuxième matériau PCM et comprise entre 25° et 30°C, de préférence entre 28° et 30°C et un deuxième matériau PCM dont la température de fusion est plus élevée que la température de fusion du premier matériau PCM et comprise entre 30° et 40°C, de préférence entre 32° et 35°C.

4. Dispositif selon l'une des revendications 1 à 3, **caractérisé en ce que** lesdites microcapsules de matériau(x) PCM sont appliquées par-dessus et/ou au sein d'un dit support constituant une couche intercalaire (2) perméable à l'air et à la vapeur d'eau, formant un tissu dénommé tissu 3D ou constituée d'un matériau fibreux non-tissé fortement poreux, ladite couche intercalaire (2) étant plus épaisse que lesdites première et deuxième pièces (1₁, 1₂), de préférence de 5 à 50 mm d'épaisseur.

5. Dispositif selon l'une des revendications 1 à 4, **caractérisé en ce qu'**il comprend en outre une feuille intercalaire (3) de matériau fibreux non-tissé, enduite au moins sur sa surface supérieure de dites microcapsules (4) de matériau(x) PCM, lesdites microcapsules présentant un diamètre de 10 à 500 µm, de préférence de 25 à 100 µm, et étant dispersées à raison de 10⁵ à 10⁷ microcapsules/cm², de préférence de 10⁶ à 5x10⁶ microcapsules/cm², ladite feuille intercalaire (3) étant plus mince que lesdites première et deuxième pièces (1₁, 1₂), de préférence présentant une densité surfacique de 100 à 200 g/cm².

6. Dispositif selon les revendications 4 et 5, **caractérisé en ce que** ladite chambre intérieure comprend une dite feuille intercalaire (3) de matériau fibreux non-tissé mince (3), enduite sur au moins sa face supérieure de dites microcapsules de matériau(x) PCM, ladite feuille intercalaire (3) étant fixée par-dessus la surface supérieure de ladite couche intercalaire (2) plus épaisse d'un matériau fibreux non-tissé (3), perméable à l'air et à la vapeur d'eau (2).

7. Dispositif selon l'une des revendications précédentes, **caractérisé en ce que** lesdits matériaux PCM sont des hydrocarbures paraffiniques comportant au moins une chaine en C-14, dont un premier matériau PCM dont- les températures de changement de phase sont situées entre 25° et 30° C, de préférence le nonadécane, et un deuxième matériau PCM dont les températures de changement de phase sont situées entre 30° et 35° C, de préférence le lycosane.

8. Dispositif selon l'une des revendications 1 à 7, **caractérisé en ce que** :
- la perméabilité à la vapeur d'eau de ladite deuxième pièce est inférieure à celle de ladite première pièce, et
- lesdits moyens d'évacuation sont desdites perforations (5), disposés par rapport au(x) dit(s) orifice(s) d'injection de façon à être apte à créer une circulation de l'air entrant dans ladite chambre (1₃) par ledit orifice d'injection (5a) et évacué de ladite chambre (1₃) par lesdits moyens d'évacuation, de préférence lesdites perforations (5), dans tout le volume de ladite chambre, lorsque ladite enveloppe est gonflée par de l'air injecté continûment sous pression par ledit orifice d'injection de manière à créer une surpression dans la dite chambre, et
- ladite deuxième pièce est sensiblement étanche à l'air entre ledit orifice d'injection et lesdites zones perforées ou poreuses perméables à l'air, ces dernières étant disposées suffisamment loin du(ou des)dit(s) orifice(s) d'injection pour que sensiblement tout le volume de ladite chambre soit parcouru par de l'air circulant entre le(s)dit(s) orifice(s) d'injection et lesdites zones perforées ou poreuses.

9. Dispositif selon l'une des revendications 1 à 8, **caractérisé en ce que** :
- ladite première pièce est constituée d'un substrat poreux ou perforé non étanche à l'eau et à l'air, ledit substrat étant enduit sur au moins une face d'une couche continue de polymère, de préférence de type polyuréthane, étanche à l'eau liquide et à l'air, et présentant des propriétés de transfert moléculaire de la vapeur d'eau, et
- ladite deuxième pièce est constituée d'un tissu enduit sur au moins une de ses faces d'une couche de polymère, de préférence de type polyuréthane, présentant des propriétés de transfert moléculaire à la vapeur d'eau, de préférence sur la face tournée du côté de l'intérieur de ladite chambre (1₃).

10. Procédé de régulation de l'humidité et de la température à la surface d'un élément de support (13) de type matelas ou coussin et au voisinage et au contact du corps d'un individu (14) y reposant, à l'aide d'un dispositif selon l'une des revendications 1 à 9, **caractérisé en ce qu'**on réalise les étapes dans lesquelles :
1) on dispose à plat ladite enveloppe (1) entre ledit élément de support (3) et ledit corps d'un individu (14), de telle sorte que ladite première pièce (1₁) soit tournée du côté du corps de l'individu et ladite deuxième pièce (1₂) soit tournée du côté dudit élément de support, et
2) on injecte de l'air sous pression dans ladite chambre (1₃) par ledit orifice d'injection (5a) à une pression et à un débit tels que ladite enveloppe reste gonflée en surpression en dépit de l'évacuation de l'air par lesdits moyens d'évacuation, de préférence desdites perforations (5) et de l'appui du corps sur ladite enveloppe, ladite surpression de l'air à l'intérieur de l'enveloppe par rapport à l'extérieur étant suffisante pour permettre une circulation de l'air dans tout le volume de la dite chambre.

## Patentansprüche

1. Vorrichtung zur Feuchtigkeits- und Temperaturregulierung (1) an der Oberfläche eines Stützelements (13) vom Typ Matratze oder Kissen sowie in der Nähe des und in Kontakt mit dem Körper(s) (14) einer darauf ruhenden Person, umfassend eine Hülle, die durch wenigstens zwei, im Bereich ihrer Umfangsränder (1a, 1b) vorzugsweise untereinander verbundene, weiterhin vorzugsweise durch Schweißen versiegelte (1c) Teile (1₁, 1₂) gebildet ist, die eine Innenkammer (1₃) definieren, wobei die beiden Teile aus einem ersten Teil (1₁), das dazu bestimmt ist, auf der Seite des Körpers der Person angeordnet zu werden, und aus einem zweiten Teil (1₂), das dazu bestimmt ist, auf der Seite des Stützelements (13) vom Typ Matratze oder Kissen angeordnet zu werden, bestehen, wobei das erste Teile aus einem Material besteht, das eine gegenüber Luft und flüssigem Wasser undurchlässige und wasserdampfdurchlässige Barriere bildet, wobei das zweite Teil aus einem wasserdampfdurchlässigen Material besteht, wobei das zweite Teil wenigstens eine Lufteinleitöffnung (5a) sowie Luftableitmittel umfasst, die luftdurchlässige perforierte oder poröse Bereiche, vorzugsweise Lochungen (5) umfassen, **dadurch gekennzeichnet, dass** die Innenkammer (1₃) Kapseln (4) aus Phasenwechselmaterial(ien) (PCM) umfasst, dessen (deren) niedrigste Kristallisationstemperatur über 25 °C, vorzugsweise über 28 °C liegt und dessen (deren) höchste Schmelztemperatur unter 40 °C, vorzugsweise unter 35 °C liegt, wobei die Kapseln in einen Träger, vorzugsweise einen aus einem gewebten oder nicht gewebten Fasermaterial bestehenden luftdurchlässigen Träger (2, 3), integriert sind und/oder an dessen Oberfläche angeordnet sind.

2. Vorrichtung nach Anspruch 1, **dadurch gekennzeichnet, dass** die Kapseln Mikrokapseln mit Durchmessern von 10 bis 500 µm, vorzugsweise 25 bis 100 µm sind, die innerhalb und/oder vorzugsweise auf der Oberseite eines luftdurchlässigen Trägers (2, 3) vorzugsweise gleichmäßig beabstandet befestigt sind.

3. Vorrichtung nach Anspruch 1 oder 2, **dadurch gekennzeichnet, dass** sie wenigstens zwei Materialien vom Typ PCM umfasst, darunter ein erstes PCM-Material, dessen Kristallisationstemperatur niedriger ist als diejenige des zweiten PCM-Materials und zwischen 25 ° und 30 °C, vorzugsweise zwischen 28 ° und 30 °C liegt, und ein zweites PCM-Material, dessen Schmelztemperatur höher ist als die Schmelztemperatur des ersten PCM-Materials und zwischen 30 ° und 40 °C, vorzugsweise zwischen 32 ° und 35 °C liegt.

4. Vorrichtung nach einem der Ansprüche 1 bis 3, **dadurch gekennzeichnet, dass** die Mikrokapseln aus PCM-Material(ien) über einen und/oder innerhalb eines Träger(s) aufgebracht werden, der eine luft- und wasserdampfdurchlässige Zwischenschicht (2) bildet, die ein als 3D-Gewebe bezeichnetes Gewebe bildet oder aus einem hochporösen nicht gewebten Fasermaterial besteht, wobei die Zwischenschicht (2) dicker ist als die ersten und zweiten Teile (1₁, 1₂), vorzugsweise eine Dicke von 5 bis 50 mm aufweist.

5. Vorrichtung nach einem der Ansprüche 1 bis 4, **dadurch gekennzeichnet, dass** sie ferner eine Zwischenfolie (3) aus nicht gewebtem Fasermaterial umfasst, die wenigstens auf ihrer Oberseite mit Mikrokapseln (4) aus PCM-Material(ien) überzogen ist, wobei die Mikrokapseln einen Durchmesser von 10 bis 500 µm, vorzugsweise von 25 bis 100 µm aufweisen und in einer Menge von 10⁵ bis 10⁷ Mikrokapseln/cm², vorzugsweise von 10⁶ bis 5x10⁶ Mikrokapseln/cm² verteilt sind, wobei die Zwischenfolie (3) dünner ist als die ersten und zweiten Teile (1₁, 1₂), vorzugsweise eine Flächendichte von 100 bis 200 g/cm² aufweist.

6. Vorrichtung nach den Ansprüchen 4 und 5, **dadurch gekennzeichnet, dass** die Innenkammer eine Zwischenfolie (3) aus dünnem nicht gewebtem Fasermaterial (3) umfasst, die auf wenigstens ihrer Oberseite mit Mikrokapseln aus PCM-Material(ien) überzogen ist, wobei die Zwischenfolie (3) über der Oberseite der dickeren Zwischenschicht (2) aus einem nicht gewebten Fasermaterial (2), die luft- und wasserdampfdurchlässig (2) ist, befestigt ist.

7. Vorrichtung nach einem der vorhergehenden Ansprüche, **dadurch gekennzeichnet, dass** die PCM-Materialien paraffinische Kohlenwasserstoffe mit wenigstens einer C-14-Kette sind, darunter ein erstes PCM-Material, dessen Phasenwechseltemperaturen zwischen 25 ° und 30 °C liegen, vorzugsweise Nonadecan, und ein zweites PCM-Material, dessen Phasenwechseltemperaturen zwischen 30 ° und 35 °C liegen, vorzugsweise Lycosan.

8. Vorrichtung nach einem der Ansprüche 1 bis 7, **dadurch gekennzeichnet, dass**:
- die Wasserdampfdurchlässigkeit des zweiten Teils geringer als die des ersten Teils ist und
- die Ableitmittel Lochungen (5) sind, die gegenüber der (den) Einleitöffnung(en) angeordnet sind, so dass sie geeignet sind, eine Zirkulation der durch die Einleitöffnung (5a) in die Kammer (1₃) eintretenden und über die Ableitmittel, vorzugsweise die Lochungen (5), aus der Kammer (1₃) abgeführten Luft in dem gesamten Volumen der Kammer zu erzeugen, wenn die Hülle mit über die Einleitöffnung ununterbrochen unter Druck eingeleiteter Luft aufgeblasen wird, um in der Kammer einen Überdruck zu erzeugen, und
- das zweite Teil zwischen der Einleitöffnung und den perforierten oder porösen, luftdurchlässigen Bereichen im Wesentlichen luftdicht ist, wobei letztere von der (den) Einleitöffnung(en) weit genug weg angeordnet sind, damit im Wesentlichen das gesamte Volumen der Kammer von zwischen der (den) Einleitöffnung(en) und den perforierten oder porösen Bereichen zirkulierender Luft durchströmt wird.

9. Vorrichtung nach einem der Ansprüche 1 bis 8, **dadurch gekennzeichnet, dass**:
- das erste Teil von einem porösen oder perforierten, nicht wasser- und luftdichtem Substrat gebildet ist, wobei das Substrat auf wenigstens einer Seite mit einer durchgehenden Polymerschicht, vorzugsweise vom Typ Polyurethan, überzogen ist, die gegenüber flüssigem Wasser und Luft dicht ist und Eigenschaften eines Molekültransports von Wasserdampf aufweist, und
- das zweite Teil aus einem Gewebe besteht, das auf wenigstens einer seiner Seiten mit einer Polymerschicht, vorzugsweise vom Typ Polyurethan, die Eigenschaften eines Wasserdampf-Molekültransports aufweist, vorzugsweise auf der der Innenseite der Kammer (1₃) zugwandten Seite, überzogen ist.

10. Verfahren zur Feuchtigkeits- und Temperaturregulierung an der Oberfläche eines Stützelements (13) vom Typ Matratze oder Kissen sowie in der Nähe des und in Kontakt mit dem Körper(s) einer darauf ruhenden Person (14), mit Hilfe einer Vorrichtung nach einem der Ansprüche 1 bis 9, **dadurch gekennzeichnet, dass** die Schritte durchgeführt werden, bei denen:
1) die Hülle (1) zwischen dem Stützelement (13) und dem Körper einer Person (14) derart flach angeordnet wird, dass das erste Teil (1₁) der Seite des Körpers der Person zugewandt ist und das zweite Teil (1₂) der Seite des Stützelements zugewandt ist, und
2) über die Einleitöffnung (5a) unter Druck stehende Luft mit einem solchen Druck und Durchsatz in die Kammer (1₃) eingeleitet wird, dass die Hülle trotz des Ableitens der Luft über die Ableitmittel, vorzugsweise Lochungen (5), und des Aufliegens des Körpers auf der Hülle, unter Überdruck aufgeblasen bleibt, wobei der Überdruck der Luft innerhalb der Hülle im Vergleich zu außerhalb ausreichend ist, um ein Zirkulieren der Luft in dem gesamten Volumen der Kammer zu ermöglichen.

## Claims

1. Device for regulating the moisture and temperature (1) on the surface of a support element (13) of mattress or cushion type and adjacent and in contact with the body (14) of an individual lying there, comprising a casing formed by at least two parts (1₁, 1₂) preferably connected together at their peripheral edges (1a, 1b), more preferably sealed by welding (1c), defining an interior chamber (1₃), said two parts consisting of a first part (1₁) intended to be placed on the side of said body of the individual, and a second part (1₂) intended to be placed on the side of said support element (13) of mattress or cushion type, said first part consisting of a material forming an impermeable barrier to air and liquid water and permeable to water vapor, said second part consisting of a material permeable to water vapor, said second part comprising at least one injection port (5a) of air and means for air evacuation comprising porous or perforated areas permeable to air, preferably perforations (5), **characterized in that** said inner chamber (1₃) comprises capsules (4) of phase change material(s) (PCM), whose lowest crystallization temperature is above 25°C, preferably above 28°C and the highest melting temperature is less than 40°C, preferably less than 35°C, said capsules being integrated within and/or placed on the surface of a support, preferably a support consisting of a woven or non-woven fibrous material permeable to air (2,3).

2. Device according to claim 1, **characterized in that** said capsules are microcapsules with diameters of 10 to 500 µm, preferably 25 to 100 µm, fixated, preferably regularly spaced, within and/or preferably on the upper surface of an air-permeable support (2,3).

3. Device according to claim 1 or 2, **characterized in that** it comprises at least two materials of the PCM type, of which a first PCM whose crystallization temperature is lower than that of the second PCM and between 25° and 30°C, preferably between 28° and 30°C and a second PCM whose melting temperature is higher than the melting temperature of the first PCM and ranging between 30° and 40°C, preferably between 32° and 35°C.

4. Device according to one of the claims 1 to 3, **characterized in that** said PCM microcapsules are applied above and/or within said support constituting an intermediate layer (2) permeable to air and water vapor, forming a fabric named 3D fabric or consisting of a fibrous non-woven very porous material, said intermediate layer (2) being thicker than the first and second parts (1₁, 1₂), preferably 5 to 50 mm thick.

5. Device according to one of the claims 1 to 4, **characterized in that** it also includes an intermediate sheet (3) of fibrous nonwoven material, coated at least on its upper surface with said PCM microcapsules (4), which microcapsules have a diameter of 10 to 500 µm, preferably 25 to 100 µm, and are distributed at the rate of 10⁵ to 10⁷ microcapsules/cm², preferably 10⁶ to 5x10⁶ microcapsules/cm², said intermediate sheet (3) being thinner than said first and second parts (1₁, 1₂), preferably presenting a surface density of 100 to 200 g/cm².

6. Device according to claims 4 and 5, **characterized in that** said interior chamber includes an intermediate sheet (3) made of thin fibrous nonwoven material (3), coated at least on its upper face with said PCM microcapsules, said intermediate sheet (3) being fixated above the upper surface of said intermediate layer (2) thicker, made of fibrous nonwoven material (3), permeable to air and to water vapor (2).

7. Device according to one of the preceding claims, **characterized in that** said PCMs are paraffinic hydrocarbons having at least one chain in C-14, of which a first PCM whose phase change temperatures are between 25° and 30°C, preferably the alkane, and a second PCM whose change phase temperatures are between 30° and 35°C, preferably the lycosane.

8. Device according to one of the claims 1 to 7, **characterized in that**:
- the permeability to water vapor of said second part is lower than that of the first part, and
- said evacuation means are said perforations (5), arranged with respect to said injection port(s) so as to be capable of creating a flow of air entering said chamber (1₃) by said injection port (5a) and evacuated from said chamber (1₃) by said evacuation means, preferably said perforations (5), throughout the volume of said chamber, when said casing is inflated by the air injected continuously pressurized through said injection port so as to create an overpressure in said chamber, and
- said second part is substantially airtight between said injection port and said porous or permeable to air perforated areas, the latter being arranged far enough away from said injection port(s) so that substantially the entire volume of said chamber is crossed by the air circulating between said injection port(s) and said porous or perforated areas.

9. Device according to one of the claims 1 to 8, **characterized in that**:
- said first part consists of a porous or perforated substrate, not impermeable to water and air, said substrate being coated on at least one face by a continuous polymer layer, preferably of the polyurethane type, impermeable to liquid water and air, and presenting molecular transfer properties of the water vapor, and
- said second part consists of a fabric coated on at least one of its sides with a layer of polymer, preferably of the polyurethane type, having molecular transfer properties to the water vapor, preferably on the face facing the side within said chamber (1₃).

10. A method of regulating the temperature and moisture on the surface of a support element (13) of the mattress or cushion type and in the vicinity and in contact with the body of an individual (14) lying there, by means of a device according to one of the claims 1 to 9, **characterized in that** the following stages are performed:
1) we arrange said casing (1) flat between said support element (13) and said body of an individual (14), so that said first part (1₁) is turned towards the body of the individual and said second part (1₂) faces the side of said support element, and
2) air is injected under pressure into said chamber (1₃) through said injection port (5a) and at a pressure and at a rate such that said casing remains inflated in overpressure despite the evacuation of the air by said evacuation means, preferably said perforations (5) and support of the body on said casing, whereby said air pressure inside the casing relative to the outside is sufficient to permit air circulation throughout the volume of said chamber.
